(19) Europäisches Patentamt
European Patent Office
Office européen des brevets

(11) Publication number: **0 018 076**
**B1**

# (12) EUROPEAN PATENT SPECIFICATION

(45) Date of publication of patent specification: **21.09.83**

(21) Application number: **80300745.9**

(22) Date of filing: **11.03.80**

(51) Int. Cl.³: **C 07 D 295/08,**
C 07 D 295/14,
C 07 D 295/00,
C 07 C 91/23,
C 07 C 103/28,
C 07 C 103/76,
C 07 C 121/78,
A 61 K 31/645

## (54) 9-Aminoalkylfluorenes, process therefor and antiarrhythmic formulations thereof.

(30) Priority: **12.03.79 US 19533**

(43) Date of publication of application:
**29.10.80 Bulletin 80/22**

(45) Publication of the grant of the patent:
**21.09.83 Bulletin 83/38**

(84) Designated Contracting States:
**DE GB NL SE**

(56) References cited:
**DE - B - 1 203 253**
**FR - A - 1 085 563**
**FR - A - 1 345 419**
**FR - A - 1 482 727**
**FR - A - 1 583 846**
**US - A - 3 660 485**

(73) Proprietor: **ELI LILLY AND COMPANY**
**307, East McCarty Street**
**Indianapolis Indiana 46285 (US)**

(72) Inventor: **Lacefield, William Bryant**
**636 Boulder Road**
**Indianapolis Indiana 46217 (US)**
Inventor: **Simon, Richard Lee**
**371 Redbud Place**
**Greenwood Indiana 46142 (US)**

(74) Representative: **Crowther, Terence Roger**
**European Patent Attorney et al,**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 018 076 B1

Courier Press, Leamington Spa, England

# 9-aminoalkylfluorenes, process therefor and antiarrhythmic formulations thereof

This invention concerns fluorenes which bear an aminoalkyl substituent at the 9-position, such compounds being useful antiarrhythmic agents. The invention also embraces pharmaceutical formulations containing the novel fluorenes as well as processes for their preparation.

Cardiovascular disorders are responsible for thousands of deaths each year. Cardiac arrhythmias are one of these disorders contributing to such deaths. While the true causes of arrhythmias are unknown, it is believed that they are caused by some abnormality in the rate, regularity or site of origin of cardiac impulses, or by disturbances which affect the sequence of activation of the atria and ventricles.

Several drugs currently are used in the treatment of arrhythmias, the most notable including quinidine, procainamide, lidocaine and digitalis. Unfortunately, most if not all of these existing drugs possesss undesirable side effects and thus there exists a continuing need for new drugs capable of being used in the treatment of arrhythmias and possessing reduced incidence of undesired side effects.

Certain 9-dialkylaminopropyl-9-hydroxyfluorenes have been previously described, see for example *J. Org. Chem., 26,* 2383—92 (1961) where such compounds were described as possessing tranquilizing properties. Reference may also be made to U.K. Patent No. 960,758 where this type of tertiary amino derivative is also described as possessing psychotherapeutic properties. French Patents Nos 1 482 727, 1 345,419, 1 583 846 and 1 085 563 and West German Patent 1 203 253 all disclose various substituted fluorene compounds of having various pharmacological properties or as being of use as intermediates. It is to be noted that those references do not teach antiarrhythmic activity for this type of compound.

USP, 3 660 485 (Searle) discloses certain specific 9-hydrazinocarbonyl substituted fluorene compounds which have antibiotic, anthelmintic, antifungal, antialgel and antiarrythmic activity. These compounds are prepared from the corresponding 9-alkoxycarbonyl substituted fluorenes, for which latter compounds, however, no pharmacological activity is suggested A later USP, 3 843 657 (Searle) discloses certain specific 9-(4-methylpiperazinocarbonyl) substituted fluorene compounds as having anti-bacterial and antifungal activity. There is no indication, however, of such compounds having any antiarrhythmic activity.

According to the present invention there is provided a 9-aminoalkylfluorene of formula (I)

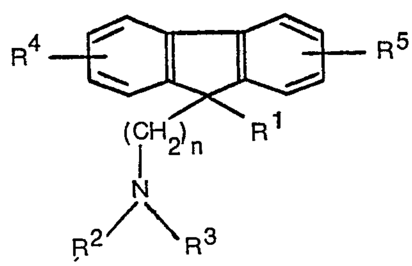

(I)

wherein:

$R^1$ is hydroxy, cyano, $CONR^6R^7$ in which $R^6$ and $R^7$ are independently hydrogen or $C_1$—$C_6$ alkyl;

n is an integer from 3 to 5;

$R^2$ and $R^3$ are independently hydrogen, $C_1$—$C_6$ alkyl, $CH_2C_2$—$C_5$ alkenyl or phenyl $C_1$—$C_3$ alkyl, or taken together with the adjacent nitrogen atom form a heterocyclic group containing from 5 to 7 ring atoms and optionally including one further hetero-atom selected from oxygen and nitrogen, and which ring is optionally substituted by up to two $C_1$—$C_4$ alkyl groups;

$R^4$ and $R^5$ are independently hydrogen, $C_1$—$C_4$ alkyl or halogen;

provided that $R^2$ is hydrogen when $R^1$ is hydroxy; or a pharmaceutically-acceptable salt thereof.

A preferred class of compounds of formula (I) are those wherein $R^1$ is hydroxy, cyano or $CONR^6R^7$ in which $R^6$ and $R^7$ are independently hydrogen or $C_1$—$C_6$ alkyl; n is 3 or 4; $R^2$ and $R^3$ are independently hydrogen, $C_1$—$C_6$ alkyl, $CH_2C_2$—$C_5$ alkenyl or phenyl $C_1$—$C_3$ alkyl, or taken together represent $C_4$—$C_5$ alkylene or $—CH_2—CH_2—O—CH_2CH_2—$; and $R^4$ and $R^5$ are hydrogen.

Other preferred compounds of formula (I) have one or more of the following features:

1. n is 3,
2. $R^1$is cyano,
3. $R^1$ is $CONH_2$,
4. $R^2$ is hydrogen and $R^3$ is $C_1$—$C_4$ alkyl,
5. $R^2$ is hydrogen and $R^3$ is *iso*propyl,
6. $R^4$ and $R^5$ are hydrogen.

The presently most preferred compound is the compound in which $R^1$ is $CONH_2$, n is 3, $R^2$ is hydrogen, $R^3$ is *iso*propyl and $R^4$ and $R^5$ are hydrogen.

As stated above also included within this invention are the pharmaceutically acceptable salts of

the compounds of the formula (I), including the quaternary ammonium salts such as those formed with $C_1$—$C_6$ alkyl alkylating agents when $R^2$ and $R^3$ are both other than hydrogen.

As used herein, the term "$C_1$—$C_6$ alkyl" refers to both straight and branched chain alkyl groups such as methyl, ethyl, *n*-propyl, *iso*-propyl, *n*-butyl, *iso*-butyl, *tert*-butyl, *n*-pentyl, *iso*-pentyl, *n*-hexyl-2-methylbutyl or 2-ethylbutyl. Preferred alkyl groups are $C_1$—$C_3$ alkyl groups such as methyl, ethyl and *iso*-propyl.

The term "$CH_2C_2$—$C_5$ alkenyl" includes unsaturated carbon chains such as allyl, 2-butenyl, 2-pentenyl, 2-hexenyl, 2-methyl-2-pentenyl, 3-hexenyl and 4-methyl-2-pentenyl. Typical examples of phenyl-$C_1$—$C_3$ alkyl groups are benzyl, 2-phenethyl and 3-phenylpropyl.

$R^2$ and $R^3$ together with the adjacent nitrogen atom may complete a heterocyclic ring containing from 5 to 7 ring atoms. Examples of such groups are the pyrrolidino, piperidino, piperazino and morpholino groups.

The compounds of formula (I) may be prepared by:

(a) reducing an aminoalkyne of formula (II):

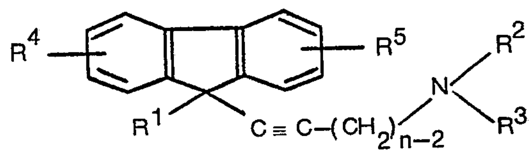

(II)

(b) reacting a ketone of formula (III):

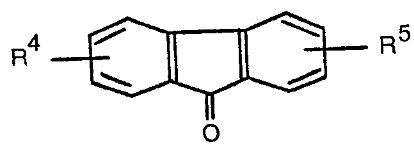

(III)

with a Grignard reagent of a formula:

$$R^2R^3N(CH_2)_n\ MgX$$

where X is chlorine, bromine or iodine, to produce a compound of formula (I) in which $R^1$ is hydroxy;

(c) alkylating a compound of formula (IV):

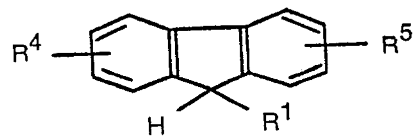

(IV)

with an aminoalkylating agent;

(d) condensing a compound of formula (V)

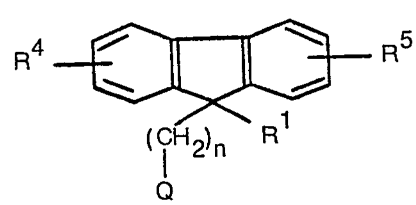

(V)

wherein Q is a leaving group, with an amine of formula $HNR^2R^3$;

(e) reacting an amine of formula (VI)

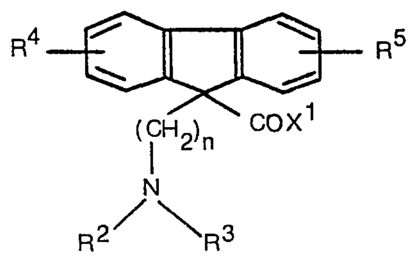

(VI)

where $X^1$ is halo or a $C_1$—$C_4$ alkyl ester group, with an amine of formula $HNR^6R^7$; or

(f) hydrolyzing a compound of formula (I) in which $R^1$ is CN to the corresponding primary amide where $R^1$ is $CONH_2$.

Compounds of formula (II) wherein $R^1$ is hydroxy can be prepared by reacting a fluoren-9-one with an alminoalkyme in the presence of a strong base which upon hydrogenation affords the corresponding 9-aminoalkyl-9-hydroxyfluorene of the invention. This process can be illustrated as follows.

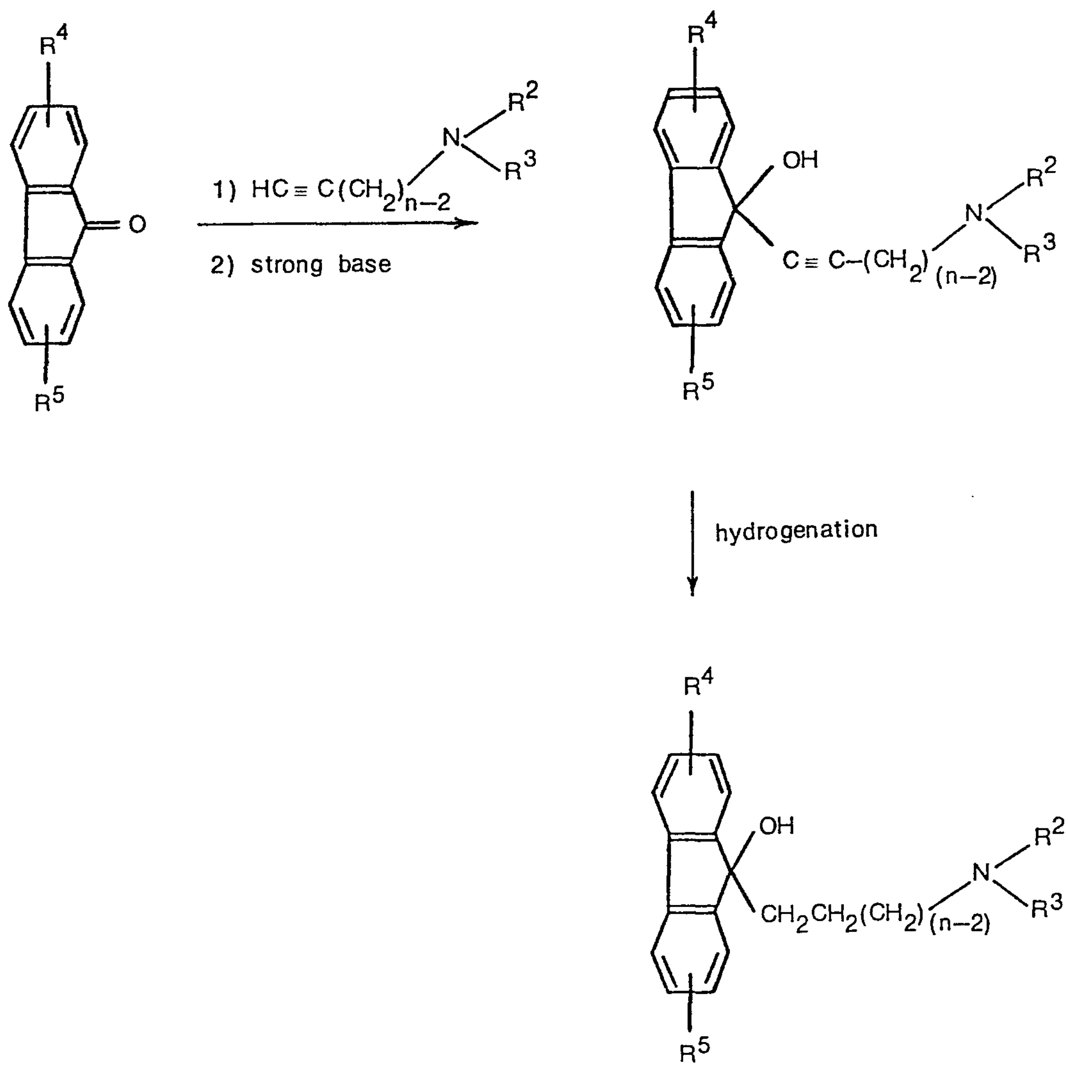


The alkynylation can be carried out by combining approximately equimolar amounts of the strong base with the aminoalkyne. Strong bases commonly utilized include alkali metal lower alkyl metalides such as methyl lithium, *n*-butyl lithium, methyl sodium; alkali metal amides such as sodium amide, potassium amide and lithium diisopropylamide, as well as alkali metal hydrides such as sodium and potassium hydride. Examples of aminoalkynes which may be used include 3-methylaminopropyne, 4-diethylaminobutyne, 4-pyrrolidinobutyne, 3-*iso*propylaminopropyne and 3-piperidinopropyne and 3-*tert*-butylaminopropyne. Typically the strong base and aminoalkyne are combined in an unreactive organic solvent such as diethyl ether, tetrahydrofuran, toluene, 1,2-dimethoxyethane or xylene and

stirred for about one hour at a reduced temperature of about —40 to about —80°C. The compound of formula II in this procedure is then added to the cold reaction mixture and stirring continued at sub-zero temperatures for about one hour, after which time the mixture is heated to about 30 to 80°C. and stirred for an additional eight to sixteen hours. After the reaction is complete, the mixture is diluted with water, thus decomposing any remaining strong base, and the product extracted into a suitable water immiscible solvent such as diethyl ether or benzene. Removal of the solvent, for instance by evaporation under reduced pressure, provides the corresponding 9-aminoalkynyl-9-($R^1$)fluorene.

The compound of formula (II) thus obtained can be converted to the corresponding 9-aminoalkyl-9-($R^1$)fluorene by hydrogenation in the presence of a catalyst such as platinum or palladium on carbon. Such hydrogenation reactions generally are carried out in an inert organic solvent such as ethyl acetate or ethanol, and normally are complete after about two to ten hours when carried out at a hydrogen pressure of about 60 psi. The reduction is preferably effected at a temperature in the range from —25° to 100°C., normally at or about room temperature. The product can be isolated by simply filtering off the catalyst and removing the reaction solvent, for example by evaporation. The product can be further purified if needed by routine methods such as distillation or salt formation.

The 9-aminoalkyl-9-hydroxyfluorenes can alternatively be prepared by reaction of fluoren-9-one with an aminoalkyl Grignard reagent, i.e. by process alternative (b). Grignard reactions are typically effected in ethereal solvents such as diethyl ether or tetrahydrofuran and at temperatures within the range from —25° to 110°C. Reaction of fluoren-9-one with a Grignard reagent such as 3-di*iso*propylaminopropyl magnesium chloride affords, after a standard work-up and isolation, the corresponding 9-aminoalkyl-9-hydroxyfluorene, namely 9-(3-di*iso*propylaminopropyl)-9-hydroxyfluorene.

The compounds of this invention can also be prepared by process variant (c), i.e. by alkylation of a compound of formula (IV) with an aminoalkyl alkylating agent. Such alkylating agents may be represented by the formula:

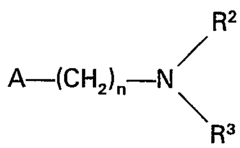

where A represents a leaving group such as chloro, bromo, iodo, azido or methanesulfonyl.

Typical alkylating agents include aminoalkyl halides such as 3-methylaminopropyl chloride, 2-*iso*-propylaminopropyl bromide, 3-diethylaminopropyl iodide, 3-ethyl*iso*butylaminopropyl chloride, 4-*iso*propylaminobutyl iodide and 3-di-*n*-hexylaminopropyl bromide. The alkylation reaction can be effected by commingling approximately equimolar quantities of the compound of formula (IV) and an aminoalkyl alkylating agent in a suitable unreactive solvent such as toluene or benzene and in the presence of about an equimolar quantity of strong base such as sodium amide, lithium amide, *n*-butyl lithium, sodium methoxide, potassium *tert*-butoxide or the like. The alkylation normally is substantially complete after about ten to twenty hours when carried out at a temperature from about 30° to about 100°Cl, although temperatures as high as 150°C. may be utilized. The alkylated fluorene can be isolated by simply diluting the reaction mixture with water and then extracting the product therefrom into a water immiscible solvent such as benzene, diethyl ether or the like. Removal of the solvent then provides the 9-cyanofluorene of this invention, which can be further purified if desired by routine methods, including distillation and salt formation. This method is particularly suited for the preparation of compounds where $R^1$ is cyano but is not always satisfactory for compounds where $R^1$ is hydroxy since such compounds tend to undergo O-alkylation.

The 9-aminoalkyl-9-cyanofluorenes which can thus be prepared are valuable antiarrhythmic agents, and additionally serve as intermediates leading to the primary 9-carboxamides of this invention, i.e. compounds of the above formula wherein $R^1$ is $CONR^6R^7$ and $R^6$ and $R^7$ both are hydrogen. The 9-aminoalkyl-9-cyanofluorenes can be hydrolyzed to the corresponding primary carboxamides by reaction with any of a number of acids such as concentrated sulfuric acid, acetic acid, boron trifluoride or dry hydrogen chloride; or alternatively by reaction with hydrogen peroxide and a base such as sodium hydroxide or with manganese dioxide in dichloromethane. The hydrolysis is conveniently effected at a temperature within the range from 20° to 110°C. A preferred hydrolysis process comprises simply heating a solution of 9-cyanofluorene in sulfuric acid for about one hour at a temperature of about 90 to 100°C. The corresponding primary carboxamide that is formed is readily isolated by making the reaction mixture alkaline, for example by adding sodium hydroxide until the pH reaches about 10, and then extracting the primary carboxamide into a suitable water immiscible solvent such as diethyl ether or benzene. Evaporation of the organic solvent then provides the desired 9-aminoalkyl-9-aminocarbonylfluorene. This compound can be further purified if desired by crystallization or salt formation.

Process variant (d), i.e. the condensation of the compound of formula (V) with the amine $HNR^2R^3$ is preferably effected by refluxing, or heating under pressure, the formula (V) compound in the presence of an acid-acceptor using excess of the amine as solvent. Q is preferably a halogen atom such as

chlorine. The compounds of formula (V) may be prepared by reacting a compound of formula (IV) with an alkylating agent of formula $Br(CH_2)_nQ$ in an inert organic solvent such as toluene or benzene.

Compounds of this invention wherein $R^1$ is $CONR^6R^7$ and one or both of $R^6$ and $R^7$ are alkyl can be prepared by process variant (e), i.e. by reacting a 9-aminoalkyl-9-fluorenyl carboxylic acid halide or lower alkyl ester with a primary or secondary amine of the formula $HNR^6R^7$. For example, a fluorenyl carboxylic acid such as 9 - (3 - N - benzyl - N - *iso*propylaminopropyl) - 9 - hydroxycarbonylfluorene can be reacted with oxalyl chloride to give the corresponding acid chloride, namely 9 - (3 - N - benzyl - N - *iso*propylaminopropyl) - 9 - chlorocarbonylfluorene. Reaction of the latter compound with an amine such as methylamine affords the corresponding methyl substituted carboxamide, namely 9 - (3 - N - benzyl - N - *iso*propylaminopropyl) - 9 - methylaminocarbonylfluorene. The substituted carboxamides of the invention can alternatively be prepared by reacting a substituted amine with an ester of a fluorene 9-carboxylic acid. This method is similar to that described in U.S. Patent 3,660,485. For example, an ester such as 9 - (3 - ethylaminopropyl) - 9 - methoxycarbonylfluorene can be reacted with an excess of an amine such as *iso*propylamine in a suitable solvent such as toluene to provide the corresponding carboxamide, namely 9 - (3 - ethylaminopropyl) - 9 - *iso*propylaminocarbonylfluorene.

Primary or secondary amines of formula (I) can be alkylated, if desired. Such a process is depicted by the following scheme:

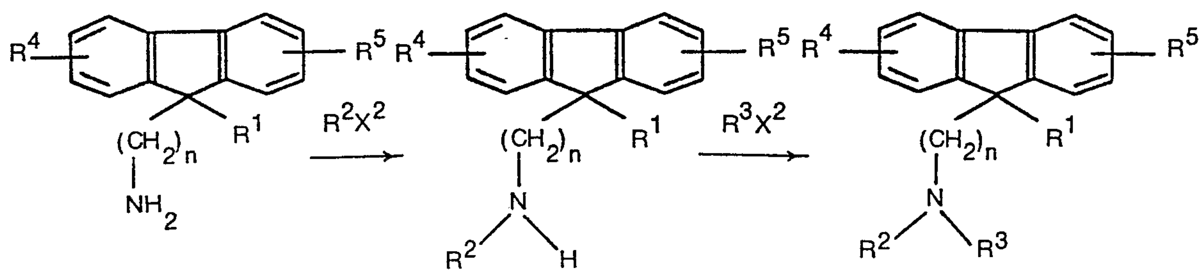


in which n, $R^1$ and $R^3$ have the above-defined meanings, and $X^2$ is a leaving group such as halogen. Alkylation of primary amines is well known in the art and typically is carried out by combining the amine and alkylating agent in the presence of a base such as sodium bicarbonate or triethylamine to act as an acid scavenger. The reactions normally are carried out in organic solvents such as toluene, dimethylsulfoxide, ethanol or methanol. As an illustration of the process, a flourene derivative such as 9-(3-aminopropyl)-9-cyanofluorene can be mixed with about an equimolar quantity of an alkylating agent such as allyl bromide in a solvent such as benzene and in the presence of triethylamine. The reaction mixture can be heated to about 50°C. and stirred for about two hours to provide the corresponding alkylated aminopropylfluorene derivative wherein $R^2$ is allyl, namely 9-(3-allylaminopropyl)-9-cyanofluorene. Further alkylation of this secondary amine with yet a different alkylating agent, for example $R^3X^2$ wherein $R^3$ is benzyl, affords the corresponding tertiary amine, namely 9 - (3 - N - allyl - N - benzylaminopropyl) - 9 - cyanofluorene. It should be at once recognized that hydrolysis of the cyano moiety as hereinabove discussed provides the corresponding 9-carboxamide derivative.

Various of the compounds provided by this invention are useful as intermediates in addition to being valuable antiarrhythmic agents. For example, the N-benzyl aminoalkylfluorenes of the above formula can be de-benzylated by hydrogenation in the presence of a catalyst such as palladium. Similarly, N-methyl aminoalkylfluorenes can be de-methylated. For example, a compound such as 9 - (4 - N - *n* - propyl - N - methylaminobutyl) - 9 - cyanofluorene can be reacted with a haloformate such as phenyl chloroformate to form a carbamate, which when reacted with alkali is hydrolyzed to the corresponding secondary amine, namely 9 - (4 - N - *n* - propylaminobutyl) - 9 - cyanofluorene. The latter compound is a potent antiarrhythmic agent, and additionally can be utilized as an intermediate in the preparation of other antiarrhythmic agents.

Ester and carboxylic acid analogues of the compounds of formula (I), i.e. compounds in which $R^1$ is $CO_2R^8$ where $R^8$ is hydrogen or $C_1$—$C_4$ alkyl, have also been found to possess antiarrhythmic properties. They can be prepared by generating the anion of a compound of formula:

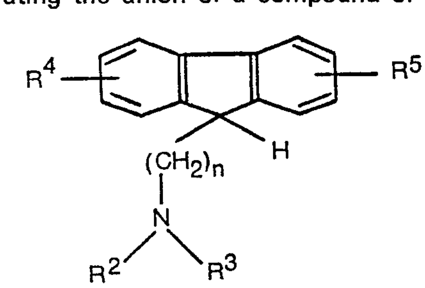

where neither of $R^2$ and $R^3$ are hydrogen, with a strong base such as sodium hydride and then reacting the anion thus formed with a $C_1$—$C_4$ alkyl haloformate, preferably a $C_1$—$C_4$ alkyl chloroformate such as ethyl chloroformate. The reaction is preferably effected in an aprotic solvent such as dimethylsulfoxide. If it is desired to prepare a compound of formula (I) in which one or both of $R^2$ and $R^3$ are hydrogen then the amino group should first be protected by readily removable groups such as methyl or benzyl, which groups are then removed to yield the desired compound. Esters of formula (I) can be hydrolyzed by aqueous bases to yield the corresponding acid ($R^1$ is COOH). These acids may also be formed by acid hydrolysis of the corresponding nitriles.

The compounds provided by this invention are basic in nature by virtue of the nitrogen atom of the 9-aminoalkyl substituent. Such compounds consequently react with a number of acids to form salts. This invention additionally provides pharmaceutically acceptable salts of the compounds defined by the above general formula, which are those salts which add no substantial toxicity to the free base from which they are derived.The pharmaceutically acceptable acid addition salts thus provided are prepared by reacting a 9-aminoalkylfluorene of this invention with any of a number of acids. Inorganic acids commonly utilized include hydrochloric, hydrobromic, phosphoric, sulfuric, nitric, perchloric and similar acids. Organic acids frequently utilized to form pharmaceutically acceptable acid addition salts include acetic succinic, maleic, methanesulfonic, citric, fumaric, *para*-toluenesulfonic, and related organic acids.

Additionally provided herein are the lower alkyl quaternary ammonium salts which can be prepared when $R^2$ and $R^3$ in the formula (I) are both other than hydrogen. For example, normal alkylation of a tertiary amine such as 9 - (3 - N - methyl - N - *iso*propylaminopropyl) - 9 - aminocarbonylfluorene by reaction with a lower $C_1$—$C_6$ alkylating agent such as methyl chloride, ethyl bromide, *n*-butyl iodide or *iso*hexyl bromide affords the corresponding quaternary ammonium salt. Such salts characteristically are highly crystalline solids and can be purified by recrystallization from solvents such as ethanol or water.

The following compounds are representative of those comprehended by this invention.

9-(3-*iso*propylaminopropyl)-9-hydroxyfluorene;
9-(4-*iso*propylaminobutyl)-9-hydroxyfluorene;
9-(3-*n*-hexylaminopropyl)-9-hydroxyfluorene;
9-(3-*tert*-butyalminopropyl)-9-hydroxyfluorene hydrobromide;
9-[3-(2-butenylamino)propyl]-9-hydroxyfluorene;
9-[4-(2-phenylethylamino)butyl]-9-hydroxyfluorene;
9-(3-*iso*propylaminopropyl)-9-cyanofluorene;
9-(3-benzylaminopropyl)-9-cyanofluorene;
9-(4-allylaminobutyl)-9-cyanofluorene;
9-[3-(3-phenylpropylamino)propyl]-9-cyanoflourene;
9-(4-N-*iso*propyl-N-ethylaminobutyl)-9-cyanofluorene;
9-(3-N,N-di*iso*propylaminopropyl)-9-cyanofluorene;
9-(3-N-*iso*propyl-N-2-phenylethylaminopropyl)-9-cyanofluorene;
9-[3-(3-hexenylamino)propyl]-9-cyanofluorene;
9-(4-diethylaminobutyl)-9-cyanofluorene;
9-(3-*iso*propylaminopropyl)-9-cyanofluorene hydrosulfate;
9-(3-dibenzylaminopropyl)-9-cyanofluorene ethyl iodide;
9-(3-*n*-pentylaminopropyl)-9-aminocarbonylfluorene;
9-(3-*iso*propylaminopropyl)-9-aminocarbonylfluorene hydrophosphate;
9-(3-N-methyl-N-*tert*-butylaminopropyl)-9-aminocarbonylfluorene;
9-[4-(2-hexenylamino)butyl]-9-aminocarbonylfluorene;
9-[3-N-(2-phenylethyl)-N-*iso*butylaminopropyl]-9-aminocarbonylfluorene;
9-(3-N,N-di-*n*-hexylaminopropyl)-9-aminocarbonylfluorene;
9-[3-N-(2-methylpentyl)-N-ethylaminopropyl]-9-aminocarbonylfluorene;
9-(3-N-benzyl-N-*iso*butylaminopropyl)-9-aminocarbonylfluorene *n*-propyl iodide;
9-(3-*iso*propylaminopropyl)-9-aminocarbonylfluorene hydroacetate;
9-(3-*iso*propylaminopropyl)-9-methylaminocarbonylfluorene;
9-(4-diethylaminobutyl)-9-dimethylaminocarbonylfluorene;
9-(3-*iso*propylaminopropyl)-9-*iso*propylaminocarbonylfluorene hydrochloride;
9-(4-benzylaminobutyl)-9-di-*n*-butylaminocarbonylfluorene;
9-(3-allylaminopropyl)-9-*n*-hexylaminocarbonylfluorene;
9-(4-morpholinobutyl)-9-aminocarbonylfluorene;
9-(3-piperidinopropyl)-9-cyanofluorene;
9-(3-aminopropyl)-9-ethylaminocarbonylfluorene;
9-(3-di*iso*propylaminopropyl)-9-di*iso*propylaminocarbonylfluorene methiodide; and
9-[3-N-(2-butenyl)-N-(2-hexenyl)aminopropyl]-9-aminocarbonylfluorene.

The 9-aminoalkylfluorenes provided by this invention are useful as antiarrhythmic agents. This utility has been demonstrated by evaluating representative compounds of the invention in biological assays designed to measure antiarrhythmic activity. One such assay comprises administering a compound of unknown biological activity to a dog suffering from an experimentally induced cardiac

arrhythmia, and observing whether or not the compound effects a conversion of the arrhythmia to a normal sinus rhythm, and if so, for how long the conversion persists.

In a typical experiment to determine the activity of the compounds of this invention, one or more mongrel dogs of either sex were anesthetized with sodium pentobarbital. A 23 gauge Butterfly infusion needle was place in the radial vein for the introduction into the dog of sufficient ouabain to induce an arrhythmia, and for the introduction into the dog of the test compound. Each dog was continuously monitored throughout the experiment by an electrocardiogram. After the ouabain induced cardiac arrhythmia had continued for thirty minutes, a compound of formula (I) was administered via the Butterfly infusion needle at the rate of 200 *m*g per kilogram of dog body weight per minute. If the arrhythmia was not converted to a normal sinus rhythm within ten minutes from the initial administration of test compound, as observed by the electrocardiogram, the rate of infusion of test compound was increased to 500 $\mu$g per kilogram per minute. The amount of test compound required to convert an arrhythmia to normal rhythm was recorded as the "converting dose". Following the complete administration of test compound to the dog, the dog's heart was monitored by electrocardiogram until such time that an arrhythmia returned to the dog's heart, or for a maximum time of two hours, at which time the experiment was terminated. The duration of normal rhythm was recorded in minutes.

The results of several experiments are set out in the following table. Most of the compounds were evaluated more than once, as indicated in the "No. of Dogs" column. The average converting dose is given in mg. per kilogram of animal body weight. Average duration of conversion is recorded in minutes.

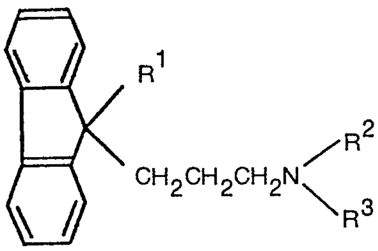

| $R^1$ | $R^2$ | $R^3$ | No. of dogs | Converting dose mg/kg | Duration minutes |
|---|---|---|---|---|---|
| $CONH_2$ | H | *i*-Pr | 3 | 0.7 | 120 |
| OH | H | i-Pr | 4 | 2.1 | 104 |
| $CONH_2$ | $CH_3$ | $CH_3$ | 1 | 3.2 | 80 |
| OH | H | $CH_2$ 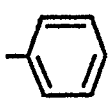 | 2 | 1.9 | 18 |

The compounds of formula (I) as well as their ester and acid analogues ($R^1$ is $CO_2R^8$ where $R^8$ is hydrogen or $C_1$—$C_4$ alkyl) can be employed in combatting cardiac arrhythmias in animals. The compounds are effective as antiarrhythmic agents when administered internally to an animal so as to introduce the compound into the animal's cardiovascular system. Parenteral administration of the compounds can be accomplished by intraperitoneal, subcutaneous or intravenous injection. The compounds alternatively can be administered orally in the form of tablets, capsules, elixirs, syrups, buccal seals and the like, and in one embodiment of the invention there is provided a pharmaceutical formulation which comprises a compound of formula (I) or an analogue in which $R^1$ is $CO_2R^8$ where $R^8$ is hydrogen or $C_1$—$C_4$ alkyl, or a pharmaceutically-acceptable satl thereof, associated with a pharmaceutically-acceptable carrier therefor.

The aminoalkylfluorenes of this invention have good antiarrhythmic activity both therapeutically, for instance when administered to an animal suffering from an arrhythmia and in need of treatment, and prophylactically when administered to an animal suspected of developing an arrhythmia, thereby protecting the animal against the occurrence or recurrence of arrhythmias.

As stated above, the compounds of the invention generally will be utilized as pharmaceutical formulations. Such formulations ideally contain from about 1 to about 50 percent by weight of the aminoalkylfluorene in combination with a suitable diluent, excipient or carrier therefor. Diluents commonly utilized in formulating the compounds in solid form suitable for oral administration include starch, lactose, gelatin, silica gel, rice flour, carboxymethyl cellulose and the like. Carriers employed in liquid formulations suitable for parenteral administration via the intravenous, intramuscular, or subcutaneous routes include water, saline, glucose syrup, ethanol, corn oil and the like.

The 9-aminoalkylfluorenes of this invention can be administered to a subject suffering from an

arrhythmia and in need of treatment, or to a subject suspected of developing an arrhythmia and in need of prophylactic treatment. Parenteral administration may be preferred for subjects suffering from a life-threatening arrhythmia. Oral administration generally is preferred for maintenance or prophylactic treatment. The compounds ideally are formulated in such a way that the effective dose of 9-aminoalkylfluorene is an amount sufficient to treat the arrhythmia. Such doses typically will be from 0.05 to 25 mg./kg. A typical oral dose for the treatment of a patient suffering from an arrhythmia will be, for example, from 1 to 200 mg. of a suitable formulated aminoalkylfluorene, for instance 9-*iso*propylaminopropyl-9-aminocarbonylfluorene, preferably as a pharmaceutically-acceptable salt such as the hydrochloride salt. Oral dosing may be made from 1 to 4 times each day, or as dictated by the particular patient and condition being treated. The fluorene can of course be formulated for parenteral administration, for instance by intravenous infusion. Such formulations can be prepared by dissolving about 500 mg. of the above-noted compound in a suitable diluent such as 1000 ml. of 5 percent glucose. The solutions thus formed can be infused at the rate of about 1 ml. per minute into a patient suffering from an arrhythmia.

The invention will now be further illustrated by the following non-limiting Examples.

Example 1

9-(3-*Iso*propylaminopropyl)-9-hydroxyfluorene

A solution of 9.0 g. of fluoren-9-one dissolved in 500 ml. of tetrahydrofuran was added dropwise over one hour to a stirred cold (—80°C.) solution of 500 ml. of tetrahydrofuran containing 9.7 g of 3-*iso*propylaminopropyne and 75 ml. of a 1.6 molar tetrahydrofuran solution of *n*-butyl lithium. After the addition was complete, the reaction mixture was warmed to room temperature and then heated to reflux for sixteen hours. The reaction mixture was next cooled to room temperature and diluted by the dropwise addition of 200 ml. of water. The aqueous mixture was extracted several times with diethyl ether, and the ethereal extracts were combined, and the reaction product was converted to the hydrochloride salt by the addition of 300 ml. of 6N hydrochloric acid to the ethereal solution. The aqueous acid layer containing the reaction product was separated, washed once with fresh diethyl ether, and then made alkaline by the addition of 10 percent sodium hydroxide, which effected liberation of the hydrochloride salt back to the free amine. The free amine was extracted into fresh diethyl ether. The ethereal extracts were combined, washed with water and dried. Removal of the solvent by evaporation under reduced pressure then afforded 2.3 g of 9 - (3 - *iso*propylaminopropynyl) - 9 - hydroxyfluorene. M.P. 214—215°C.

A solution of 2.3 g. of 9-(3-*iso*propylaminopropynyl)-9-hydroxyfluorene in 200 ml. of ethanol containing 3.0 g of five percent palladium on carbon was agitated at room temperature for six hours under a hydrogen atmosphere of 60 psi. The reaction mixture then was filtered to remove the catalyst, and the filtrate was concentrated to dryness by evaporation of the solvent under reduced pressure. The solid that was thus obtained was crystallized from ethyl acetate and Skelly B to afford 1.6 g. of 9-(3-*iso*propylaminopropyl)-9-hydroxyfluorene, M.P. 135—137°C.

Analysis calc. for $C_{19}H_{23}NO$

| | | | |
|---|---|---|---|
| Theory: | C, 81.10; | H, 8.24; | N, 4.98. |
| Found: | C, 81.17; | H, 8.38; | N, 4.72. |

Example 2

9-(3-Dimethylaminopropyl)-9-cyanofluorene

To a stirred solution of 2.6 g. of sodium amide in 200 ml. of tetrahydrofuran was added dropwise over thirty minutes a solution of 12.7 g. of 9-cyanoflurene in 300 ml. of tetrahydrofuran. The reaction mixture was next heated to reflux for three hours, and then cooled to room temperature. While the reaction mixture was being stirred at room temperature, a solution of 14.2 g. of 3-dimethylaminopropyl chloride in 500 ml. of tetrahydrofuran was added dropwise over one hour. Following complete addition, the reaction mixture was again heated to reflux and stirred for sixteen hours. After cooling the mixture to room temperature, it was added to 500 ml. of water. The product was extracted into diethyl ether, and the ethereal extracts were combined, washed with water and dried. Removal of the solvent by evaporation under reduced pressure afforded, after distillation, 2.3 g. of 9-(3-dimethylaminopropyrl)-9-cyanofluorene. B.P. 195—201°C. at 0.1 torr.

Examples 3—6

The following 9-aminoalkyl-9-cyanofluorenes were prepared by reacting 9-cyanofluorene with the appropriate aminoalkyl halide according to the procedure of Example 2.

9-(3-Piperidinopropyl)-9-cyanofluorene. B.P. 200—208°C. at 0.18 torr.

9-(3-Diethylaminopropyl)-9-cyanofluorene. B.P. 182—195°C. at 0.18 torr.

9-(3-N-Benzyl-N-*iso*propylaminopropyl)-9-cyanofluorene. B.P. 220—245°C. at 0.18 torr.

9-(3-*Iso*propylaminopropyl)-9-cyanofluorene.

Example 7

9-(3-*Iso*propylaminopropyl)-9-aminocarbonylfluorene

A solution of 2.5 g of 9-(3-*iso*propylaminopropyl)-9-cyanofluorene in 20 ml. of concentrated sulfuric acid and 8 ml. of water was heated at 100°C. for forty-five minutes. The reaction mixture then was added to 50 g. of ice, and 10 percent aqueous sodium hydroxide was added to pH=10. The alkaline mixture was extracted several times with diethyl ether. The ethereal extracts were combined, washed with water and dried. Removal of the solvent by evaporation under reduced pressure provided a white solid, which after crystallization from Skelly B afforded 1.2 g of 9 - (3 - *iso*propylamino-propyl) - 9 - aminocarbonylfluorene. M.P. 94—95°C.

Analysis calc. for $C_{20}H_{24}N_2O$

| | | | |
|---|---|---|---|
| Theory: | C, 77.89; | H, 7.84; | N, 9.08. |
| Found: | C, 78.17; | H, 7.65; | N, 9.00. |

Example 8—11

The following fluorene carboxamides were prepared by acid hydrolysis of the corresponding fluorene nitriles according to the procedure of Example 7.

9-(3-Piperidinopropyl)9-aminocarbonylfluorene. M.P. 155—156.5°C.

Analysis calc. for $C_{22}H_{26}N_2O$

| | | | |
|---|---|---|---|
| Theory: | C, 79.00; | H, 7.84; | N, 8.38. |
| Found: | C, 78.73; | H, 7.70; | N, 8.14. |

9-(3-Dimethylaminopropyl)-9-aminocarbonylfluorene. M.P. 91—92°C.

Analysis calc. for $C_{19}H_{22}N_2O$

| | | | |
|---|---|---|---|
| Theory: | C, 77.52; | H, 7.53; | N, 9.52. |
| Found: | C, 77.51; | H, 7.50; | N, 9.29. |

9-(3-Diethylaminopropyl)-9-aminocarbonylfluorene. M.P. 78—79°C.

Analysis calc. for $C_{20}H_{26}N_2O$

| | | | |
|---|---|---|---|
| Theory: | C, 78.22; | H, 8.13; | N, 8.69. |
| Found: | C, 78.43; | H, 8.11; | N, 8.59. |

9-(3-N-Benzyl-N-*iso*propylaminopropyl)-9-aminocarbonylflourene.

Example 12

9-(3-N-Benzyl-N-*iso*propylaminopropyl)-9-cyanofluorene (27.4 g.) dissolved in ethanol was hydrogenated by treatment for 16 hours with hydrogen (60 psi) at 40°C. in the presence of 3.0 g. of five percent palladium on charcoal to give 23.4 g. of 9-(3-*iso*propylaminopropyl)-9-cyanofluorene. M+ 290; theory 290.

Example 13

9-(3-*Iso*propylaminopropyl)-9-aminocarbonylfluorene hydrochloride.

Hydrogen chloride was bubbled into a solution of 2.357 g. of 9-(3-*iso*propylaminopropyl)-9-aminocarbonylflourene in 100 ml. of ethanol until the solution was saturated. The reaction solvent then was removed by evaporation under reduced pressure, leaving the product as an oil. The oil was crystallized from fresh ethanol and diethyl ether to give 2.145 g. of 9-(3-*iso*propylaminopropyl)-9-aminocarbonylfluorene hydrochloride. M.P. 203—204°C.

Analysis calc. for $C_{20}H_{25}N_2OCl$

| | | | |
|---|---|---|---|
| Theory: | C, 69.65; | H, 7.31; | N, 8.12. |
| Found: | C, 69.94; | H, 7.58; | N, 8.39. |

Example 14

9-(3-N-*Iso*propyl-N-methylaminopropyl)-9-hydroxyfluorene methiodide.

A solution of 2.7 g. of 9-(3-N-*iso*propyl-N-methylaminopropyl)-9-hydroxyfluorene in 20 ml. of ethanol containing 1.1 g. of methyliodide was stirred at ambient temperature for thirty minutes. The precipitated solid was collected by filtration and recrystallized from ethanol to afford 2.5 g. of 9 - (3 - N - *iso*propyl-N-methylaminopropyl) - 9 - hydroxyfluorene methiodide. M.P. 217—219°C.

Analysis calc for $C_{21}H_{28}NOI$

| | | | |
|---|---|---|---|
| Theory: | C, 57.67; | H, 6.45; | N, 3.20. |
| Found: | C, 57.65; | H, 6.30; | N, 3.22. |

Example 15

Following the procedure of Example 14, 9 - (3 - N - *iso*propyl - N - methylaminopropyl) - 9 - aminocarbonylfluorene was reacted with allyl bromide in dichloromethane to give the corresponding quaternary ammonium salt, namely 9 - (3 - N - allyl - N - *iso*propylaminopropyl) - 9 - aminocarbonylfluorene methyl bromide.

Example 16

9-(4-N-Ethyl-N-*iso*propylaminobutyl)-9-ethylaminocarbonylfluorene

To a stirred solution of 9 - (4 - N - ethyl - N - *iso*propylaminobutyl) - 9 - hydroxycarbonylfluorene in benzene was added oxalyl chloride. The reaction mixture was stirred for several hours and then the solvent then evaporated to give 9 - (4 - N - ethyl - N - *iso*propylaminobutyl) - 9 - chlorocarbonylfluorene as an oil. The oil thus formed was dissolved in dichloromethane containing triethylamine and stirred while ethylamine was added in one portion. After stirring the reaction mixture several hours, the solvent was removed to provide the corresponding N-alkyl amide, namely 9-(4-N-ethyl-N-*iso*propylaminobutyl) - 9 - ethylaminocarbonylfluorene.

Example 17

9-(3-Isopropylaminopropyl)-9-aminocarbonylfluorene

A solution of 9-(3-aminopropyl)-9-aminocarbonylfluorene in benzene containing one equivalent of *n*-isopropyl bromide and triethylamine is stirred for several hours. Removal of the reaction solvent and distillation of the product affords 9 - (3 - *n* - isopropylaminopropyl) - 9 - aminocarbonylfluorene, m.p. 94—95°C.

Example 18

9-[3-(2,6-Dimethylpiperidino)propyl]-9-cyanofluorene

(a) 2,6-Dimethyl-1-(3-chloropropyl)piperidine, hydrochloride

2,6-Dimethylpiperidine (169.5 g., 1.5 mole) was reacted under a nitrogen atmosphere with 3-bromopropanol (195 g., 1.4 mole) in 800 ml. dry tetrahydrofuran as solvent to give 2,6-dimethyl-1-(3-hydroxypropyl)piperidine. This liquid (95.2 g., 0.55 mole) was then chlorinated with thionyl chloride (178.5 g., 1.5 mole) using chloroform (1 litre) as solvent and in the presence of HCl gas to yield the title product as gray crystals (67.8 g.) m.p. 169—171°C.

(b) 9-[3-(2,6-Dimethylpiperidino)propyl]-9-cyanofluorene

9-Cyanofluorene (19.1 g., 0.1 mole) and sodamide (4.7 g., 0.12 mole) were refluxed in dry toluene in a 1 litre 3-necked flask for two hours. 2,6-Dimethyl-1-(3-chloropropyl)piperidine (22.7 g; 0.12 mole) also in dry toluene was then added to the flask and the mixture refluxed overnight. The reaction mixture was then cooled, water added dropwise and the mixture then poured into iced water and extracted with diethyl ether. After further conventional work-up and recrystallization from Skelly B, light brown crystals of the title product (22.2 g.) m.p. 78—81°C. were obtained.

Example 19

9-[3-(2,6-Dimethylpiperidino)propyl]-9-aminocarbonylfluorene, maleic acid salt

The nitrile (5.0 g.) of Example 18 was added to a 100 ml. round-bottomed flask. Sulfuric acid (90%, 20 g.) was cooled and added slowly to the nitrile in the flask. The mixture was then heated on a steam cone for 45 minutes. The solution thus formed is added to ice-water and basified with aqueous sodium hydroxide (10%). This material is then extracted with ethyl acetate and diethyl ether and the extracts washed with water. After drying ($Na_2SO_4$) the amide (5.4 g., 0.015 mole) thus formed was reacted with maleic acid (1.7 g., 0.015 mole) in ethyl acetate (150 ml) and ethanol (25 ml) in a 500 ml. single neck round-bottomed flask. The salt thus formed was recrystallized from ethanol and ethyl acetate, yield 1.3 g., m.p. 182—184°C.

Example 20

9-(4-Isopropylaminopropyl)-9-cyanofluorene, hydrochloride

(a) 9-(3-Chloropropyl)-9-cyanofluorene

9-Cyanofluorene (19.1 g., 0.1 mole) and sodamide (4.3 g., 0.11 mole) were refluxed together for 2 hours in a 1 litre flask using toluene as a solvent. 3-Bromopropyl chloride (17.3 g., 0.11 mole) in toluene was then added and the reaction mixture refluxed overnight. After work-up and recrystallization from ethyl acetate and Skelly B, the title compound was obtained as a solid. m.p. 75—77°C.

(b) 9-(4-*Iso*propylaminopropyl)-9-cyanoflurene, hydrochloride

9-(3-Chloropropyl)-9-cyanofluorene (67.5 g.) was reacted with *iso*propylamine (300 g.) at a temperature of 160°C. in the presence of potassium iodide (4 g.). The reaction mixture was concentrated, added to ice water and acidified with 6N hydrochloric acid. After conventional work-up the title product was obtained as a solid, m.p. 183—186°C., yield 35.2 g.

Example 21

9-(3-N-Benzyl-N-*iso*propylaminopropyl)-9-hydroxyfluorene

Magnesium metal (19.2 g, 0.8 mole) together with a small amount of iodine was added to a 3-litre flask under a nitrogen atmosphere. These components were stirred together with heating for 15 minutes to drive off any moisture present. The flask was then cooled to room temperature and magnesium covered with dry tetrahydrofuran, followed by the addition of a little 1,2-dibromoethane. 3-(N-Benzyl-N-*iso*propylamino)propyl chloride (62.5 g., 0.25 mole) dissolved in dry tetrahydrofuran (280 ml) was then rapidly added and the reaction mixture refluxed for about one hour. The reaction mixture was then cooled to 50°C. and fluoren-9-one (30.6 g., 0.18 mole) dissolved in dry tetrahydrofuran (200 ml) added dropwise over 10 minutes. The reactants were then refluxed for 1 hour, allowed to cool to room temperature and left standing over a weekend. After standard work-up there was obtained an oil. b.p. 200°—220°C./0.2 mm which crystallized to a solid. m.p. 68° to 70°C.

Example 22

The product of Example 21 was hydrogenolyzed by the process of Example 12 to yield 9-(3-isopropylaminopropyl)-9-hydroxyfluorene, m.p. 135—137°C.

Example 23

Similarly prepared by the process of Example 21 and 22 was: 2,7-dimethyl-9-(3-*iso*propylamino-propyl)-9-hydroxyfluorene.

Example 24

9-(3-N-Benzyl-N-*iso*propylaminopropyl)-9-ethoxycarbonylfluorene

Sodium hydride (8.6 g., 0.18 mole) was dissolved in dimethylacetamide (100 ml.) of spectrophotometric grade in a 1 litre flask. Fluorene (40 g., 0.18 mole) dissolved in dimethylacetamide (100 ml.) was then added dropwise over a period of 60 minutes at 25°C. The reaction mixture was then stirred for another 25 minutes and 3-(N-Benzyl-N-isopropylamino)propyl chloride (40.5 g., 0.18 mole) dissolved in dimethylacetamide (30 ml.) was added dropwise at 20°C. over a period of 35 minutes and the reaction mixture stirred overnight. After standard work-up, there was obtained 9-(3-N-Benzyl-N-*iso*propylaminopropyl)fluorene.

Sodium hydride (1.96 g., 0.04 mole) was dissolved in dimethylsulfoxide (200 ml.) in a 1 litre flask. This mixture was cooled to 17°C. with an ice bath and 9-(3-N-benzyl-N-*iso*propylaminopropyl)-fluorene (14.7 g., 0.041 mole) dissolved in dimethylsulfoxide (50 ml.) added dropwise over a period of 40 minutes. This mixture was then heated to 105°C. and cooled to 15°C. Ethyl chloroformate (4.42 g., 0.04 mole) was then added dropwise over a period of 15 minutes. The reactants were then stirred for 1/2 hour, heated to 105°C. for 15 minutes and stirred overnight. After standard work-up there was obtained the title product, b.p. 237°/0.3 mm yield 25.1 g.

Example 25

9-(2-N-*Iso*propylaminopropyl)-9-ethoxycarbonylfluorene, hydrochloride

The benzyl group of the product of Example 24 (9.6?g. 0.022 mole) was removed by hydrogenolysis of that material dissolved in ethanol (200 ml.) in the presence of 5% pd/c (2 g.). The hydrochloride salt was formed by forming an ethereal solution (diethyl ether) and bubbling in HCl gas. The title product crystallized out as a solid, m.p. 177—179°C. (acetone).

Example 26

| Tablet | Mg. |
|---|---|
| 9-(3-isopropylaminopropyl)-9-aminocarbonylfluorene hydrochloride | 20 |
| starch | 240 |
| sucrose | 240 |
| | 500 |

The above ingredients were throughly mixed with a lubricant and the mixture molded into a tablet.

Example 27

Formulation suitable for intravenous administration.

| Ingredient | |
|---|---|
| 9-(3-isopropylaminopropyl)-9-aminocarbonylfluorene, hydrochloride | 225 mg. |
| isotonic saline | 450 ml. |
| 10% aqueous glucose | 450 ml. |

The above ingredients were mixed to form a solution capable of being infused into a subject suffering from an arrhythmia.

**Claims**

1. A 9-aminoalkylfluorene of formula (I)

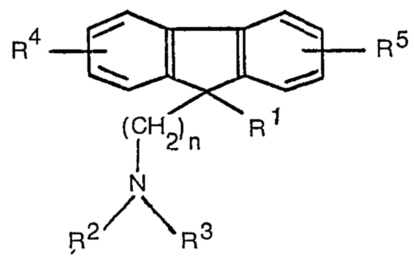

(I)

wherein:

$R^1$ is hydroxy, cyano, $CONR^6R^7$ in which $R^6$ and $R^7$ are independently hydrogen or $C_1$—$C_6$ alkyl;

n is an integer from 3 to 5;

$R^2$ and $R^3$ are independently hydrogen, $C_1$—$C_6$ alkyl, $CH_2C_2$—$C_5$ alkenyl or phenyl $C_1$—$C_3$ alkyl, or taken together with the adjacent nitrogen atom form a heterocyclic group containing from 5 to 7 ring atoms and optionally including one further heteroatom selected from oxygen and nitrogen, and which ring is optionally substituted by up to two $C_1$—$C_4$ alkyl groups;

$R^4$ and $R^5$ are independently hydrogen, $C_1$—$C_4$ alkyl groups;

$R^4$ and $R^5$ are independently hydrogen, $C_1$—$C_4$ alkyl or halogen;

provided that $R^2$ is hydrogen when $R^1$ is hydroxy; or a pharmaceutically-acceptable salt thereof.

2. A compound of formula (I) as claimed in claim 1, wherein $R^1$ is hydroxy, cyano or $CONR^6R^7$ in which $R^6$ and $R^7$ are independently hydrogen or $C_1$—$C_6$ alkyl; n is 3 or 4, $R^2$ and $R^3$ are independently hydrogen, $C_1$—$C_6$ alkyl, $CH_2C_2$—$C_5$ alkenyl or phenyl $C_1$—$C_3$ alkyl, or taken together represent $C_4$—$C_5$ alkylene or —$CH_2CH_2$—O—$CH_2CH_2$—; and $R^4$ and $R^5$ are hydrogen.

3. A compound of formula (I) as claimed in claim 1 or 2, wherein n is 3.

4. A compound of formula (I) as claimed in any one of claims 1 to 3, wherein $R^1$ is $CONH_2$.

5. A compound of formula (I) as claimed in any one of claims 1 to 4, wherein $R^2$ is hydrogen and $R^3$ is isopropyl.

6. A compound of formula (I) as claimed in any of claims 1 to 5, wherein $R^4$ and $R^5$ are hydrogen.

7. 9-(3-Isopropylaminopropyl)-9-aminocarbonylfluorene, or a pharmaceutically-acceptable salt thereof.

8. A process for preparing a compound of formula (I) which comprises:

(a) reducing an aminoalkyne of formula (II):

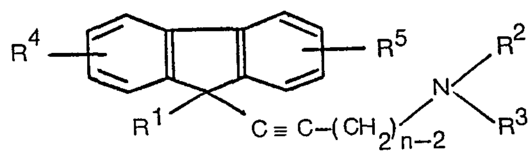

(II)

(b) reacting a ketone of formula (III):

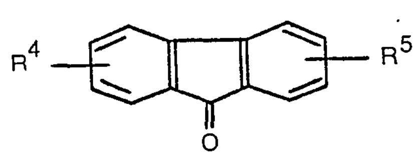

(III)

with a Grignard reagent of formula:

$$R^2R^3N(CH_2)_nMgX$$

where X is chlorine, bromine or iodine, to produce a compound of formula (I) in which $R^1$ is hydroxy;

(c) alkylating a compound of formula (IV):

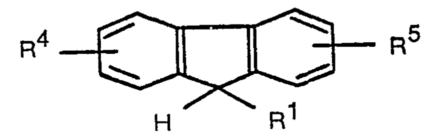

(IV)

with an aminoalkylating agent;

(d) condensing a compound of formula (V)

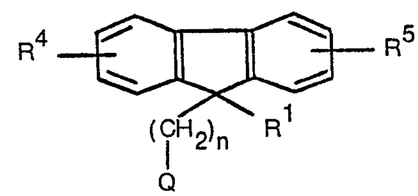

(V)

where Q is a leaving group, with an amine of formula $HNR^2R^3$;

(e) reacting an amine of formula (VI)

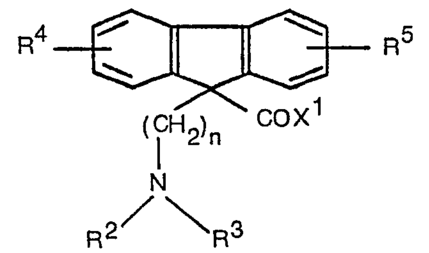

(VI)

where $X^1$ is halo or a $C_1$—$C_4$ alkyl ester group, with an amine of formula $HNR^6R^7$; or

(f) hydrolyzing a compound of formula (I) in which $R^1$ is CN to the corresponding primary amide where $R^1$ is $CONH_2$.

9. A pharmaceutical formulation which comprises as an active ingredient a compound of formula (I) in which $R^1$ is as defined in claim 1 or is —$CO_2R^8$, where $R^8$ is hydrogen or $C_1$—$C_4$ alkyl, or a pharmaceutically-acceptable salt thereof, associated with a pharmaceutically-acceptable carrier therefor.

10. A compound of formula (I) in which $R^1$ is as defined in claim 1 or is —$CO_2R^8$, where $R^8$ is hydrogen or $C_1$—$C_4$ alkyl, or a pharmaceutically-acceptable salt thereof, for use in the treatment of cardiac disorders.

**Patentansprüche**

1. 9-Aminoalkylfluoren der Formel (I)

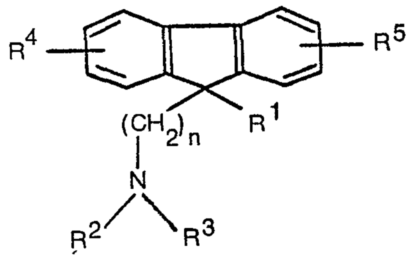

(I)

worin
$R^1$ Hydroxy, Cyano oder $CONR^6R^7$ bedeutet, worin $R^6$ und $R^7$ unabhängig Wasserstoff oder $C_1$—$C_6$-Alkyl sind,
n für eine Zahl von 3 bis 5 steht,
$R^2$ und $R^3$ unabhängig Wasserstoff, $C_1$—$C_6$-Alkyl, $CH_2C_2$—$C_5$-Alkenyl oder Phenyl-$C_1$—$C_3$-Alkyl sind, oder zusammen mit dem benachbarten Stickstoffatom eine heterocyclische Gruppe mitt 5 bis 7 Ringatomen und gegebenenfalls Sauerstoff oder Stickstoff als weiterem Heteroatom bilden, wobei dieser Ring gegebenenfalls mit bis zu zwei $C_1$—$C_4$-Alkylgruppen substituiert ist, und
$R^4$ sowie $R^5$ unabhängig Wasserstoff, $C_1$—$C_4$-Alkyl oder Halogen bedeuten,
mit der Maßgabe, daß $R^2$ für Wasserstoff steht, falls $R^1$ Hydroxy ist,
oder ein pharmazeutisch unbedenkliches Salz hiervon.

2. Verbindung der Formel (I) gemäß Anspruch 1, dadurch gekennzeichnet, daß $R^1$ Hydroxy, Cyano oder $CONR^6R^7$ ist, worin $R^6$ und $R^7$ unabhängig Wasserstoff oder $C_1$—$C_6$-Alkyl sind, n für 3 oder 4 steht, $R^2$ und $R^3$ unabhängig Wasserstoff, $C_1$—$C_6$-Alkyl, $CH_2C_2$—$C_5$-Alkenyl oder Phenyl-$C_1$—$C_3$-Alkyl sind oder zusammen für $C_4$—$C_5$-Alkylen oder —$CH_2CH_2$—O—$CH_2CH_2$— stehen und $R^4$ sowie $R^5$ Wasserstoff bedeuten.

3. Verbindung der Formel (I) gemäß Anspruch 1 oder 2, dadurch gekennzeichnet, daß n für 3 steht.

4. Verbindung der Formel (I) gemäß einem der Anspüche 1 bis 3, dadurch gekennzeichnet, daß $R^1$ für $CONH_2$ steht.

5. Verbindung der Formel (I) gemäß einem der Ansprüche 1 bis 4, dadurch gekennzeichnet, daß $R^2$ Wasserstoff ist und $R^3$ für Isopropyl steht.

6. Verbindung der Formel (I) gemäß einem der Ansprüche 1 bis 5, dadurch gekennzeichnet, daß $R^4$ und $R^5$ Wasserstoff sind.

7. 9-(3-Isopropylaminopropyl)-9-aminocarbonylfluoren oder ein pharmazeutisch unbedenkliches Salz hiervon.

8. Verfahren zur Herstellung einer Verbindung der Formel (I), dadurch gekennzeichnet, daß man
(a) ein Aminoalkin der Formel (II)

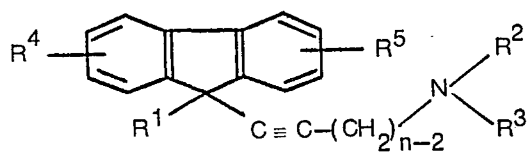

(II)

reduziert,
(b) ein Keton der Formel (III)

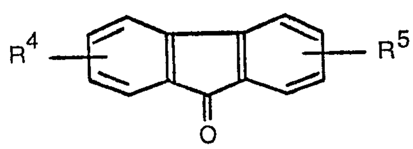

(III)

mit einem Grignard-Reagens der Formel

$$R^2R^3N(CH_2)_n\ MgX,$$

worin X Chlor, Brom oder Iod bedeutet, zu einer Verbindung der Formel (I), worin $R^1$ Hydroxy ist, umsetzt,
(c) eine Verbindung der Formel (IV)

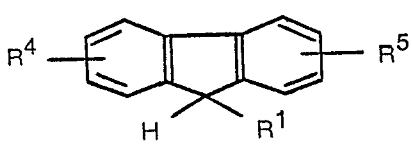

(IV)

mit einem Aminoalkylierungsmittel alkyliert,

(d) eine Verbindung der Formel (V)

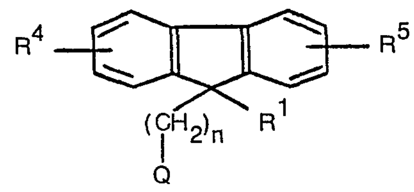

(V)

worin Q eine abspaltbare Gruppe ist, mit einem Amin der Formel

$$HNR^2R^3$$

kondensiert,

(e) ein Amin der Formel (VI)

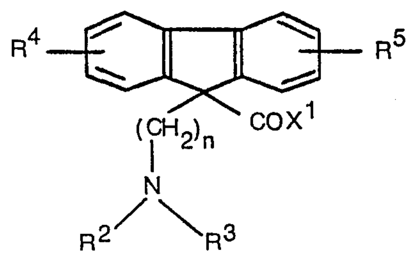

(VI)

worin $X^1$ für Halogen oder eine $C_1$—$C_4$-Alkylestergruppe steht, mit einem Amin der Formel

$$HNR^6R^7$$

umsetzt oder

(f) eine Verbindung der Formel (I), worin $R^1$ für CN steht, zum entsprechenden primären Amid, worin $R^1$ für $CONH_2$ steht, hydrolysiert.

9. Pharmazeutische Formulierung aus einer Verbindung der Formel (I), worin $R^1$ die in Anspruch 1 angegebene Bedeutung hat oder für —$CO_2R^8$ steht, worin $R^8$ Wasserstoff oder $C_1$—$C_4$-Alkyl ist, oder einem pharmazeutisch umbedenklichen Salz hiervon als Wirkstoff in Verbindung mit einem pharmazeutisch unbedenklichen Träger hierfür.

10. Verwendung einer Verbindung der Formel (I), worin $R^1$ die in Anspruch 1 angegebene Bedeutung hat oder für —$CO_2R^8$ steht, worin $R^8$ Wasserstoff oder $C_1$—$C_4$-Alkyl ist, oder eines pharmazeutisch unbedenklichen Salzes hiervon zur Behandlung von Herzstörungen.

**Revendications**

1. 9-amino-alkylfluorène de formule (I):

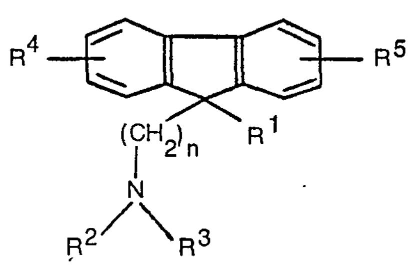

(I)

dans laquelle

$R^1$ représente un groupe hydroxy, un groupe cyano, un groupe $CONR^6R^7$ dans lequel R et $R^7$ représentent chacun indépendamment l'un de l'autre un atome d'hydrogène ou un groupe alkyle en $C_1$—$C_6$;

n est un nombre entier de 3 à 5;

$R^2$ et $R^3$ représentent chacun indépendamment l'un de l'autre un atome d'hydrogène, un groupe alkyle en $C_1$—$C_6$, un groupe $CH_2$-alcényle en $C_2$—$C_5$ ou un groupe phényl-alkyle en $C_1$—$C_3$; ou, pris ensemble avec l'atome d'azote adjacent, ils forment un groupe hétérocyclique contenant 5 à 7

atomes cycliques et comportant éventuellement un autre hétéro-atome choisi parmi l'oxygène et l'azote, ce noyau étant éventuellement substitué par un ou deux groupes alkyle en $C_1$—$C_4$;

$R^4$ et $R^5$ représentent chacun indépendamment l'un de l'autre un atome d'hydrogène, un groupe alkyle en $C_1$—$C_4$ ou un atome d'halogène;

avec cette restriction que $R^2$ est un atome d'hydrogène lorsque $R^1$ est un groupe hydroxy; ou un sel pharmaceutiquement acceptable de ce composé.

2. Composé répondant à la formule (I) suivant la revendication 1, caractérisé en ce que $R^1$ est un groupe hydroxy, un groupe cyano ou un groupe $CONR^6R^7$ dans lequel $R^6$ et $R^7$ représentent chacun indépendamment l'un de l'autre un atome d'hydrogène ou un groupe alkyle en $C_1$—$C_6$; n est égal à 3 ou 4, $R^2$ et $R^3$ représentent chacun indépendamment l'un de l'autre un atome d'hydrogène, un groupe alkyle en $C_1$—$C_6$, un groupe $CH_2$-alcényle en $C_2$—$C_5$ ou un groupe phénylalkyle en $C_1$—$C_3$ ou, pris ensemble, ils représentent un groupe alkylène en $C_4$—$C_5$ ou un groupe $—CH_2CH_2—O—CH_2—CH_2—$, tandis que $R^4$ et $R^5$ représentent chacun un atome d'hydrogène.

3. Composé répondant à la formule (I) suivant la revendication 1 ou 2, caractérisé en ce que n est égal à 3.

4. Composé répondant à la formule (I) suivant l'une quelconque des revendications 1 à 3, caractérisé en ce que $R^1$ est un groupe $CONH_2$.

5. Composé répondant à la formule (I) suivant l'une quelconque des revendications 1 à 4, caractérisé en ce que $R^2$ est un atome d'hydrogène et $R^3$ est un groupe isopropyle.

6. Composé répondant à la formule (I) suivant l'une quelconque des revendications 1 à 5, caractérisé en ce que $R^4$ et $R^5$ représentent chacun un atome d'hydrogène.

7. 9-(3-isopropylaminopropyl)-9-aminocarbonylfluorène ou un de ses sels pharmaceutiquement acceptables.

8. Procédé de préparation d'un composé de formule (I), caractérisé en ce qu'il comprend les étapes qui consistent à:

(a) réduire un amino-alcyne de formule (II):

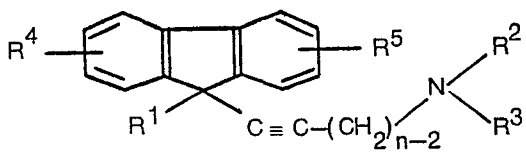

(II)

(b) faire réagir une cétone de formule (III):

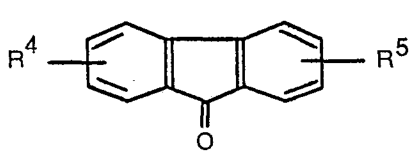

(III)

avec un réactif de Grignard de formule:

$$R^2R^3N(CH_2)_n\ MgX$$

dans laquelle X représente un atome de chlore, un atome de brome ou un atome d'iode, pour obtenir un composé de formule (I) dans laquelle $R^1$ est un groupe hydroxy;

(c) soumettre un composé de formule (IV):

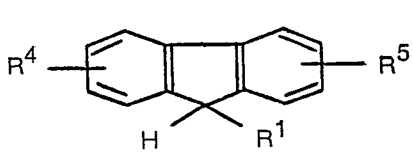

(IV)

à une alkylation avec un agent d'amino-alkylation;

(d) condenser un composé de formule (V):

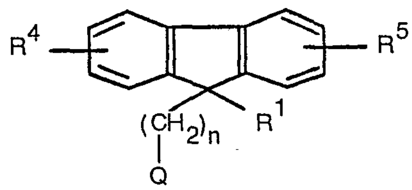

(V)

dans laquelle Q est un groupe qui s'éloigne, avec une amine de formule $HNR^2R^3$;

(e) faire réagir une amine de formule (VI):

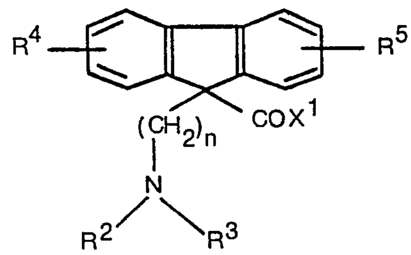

(VI)

dans laquelle $X^1$ représente un groupe halo ou un groupe d'ester alkylique en $C_1$—$C_4$, avec une amine de formule $HNR^6R^7$; ou

(f) hydrolyser un composé de formule (I) dans laquelle $R^1$ représente CN, en amide primaire correspondant dans lequel $R^1$ est un groupe $CONH_2$.

9. Formulation pharmaceutique, caractérisée en ce qu'elle comprend, comme ingrédient actif, un composé de formule (I) dans laquelle $R^1$ a la signification définie dans la revendication 1 ou représente un groupe —$CO_2R^8$ dans lequel $R^8$ est un atome d'hydrogène ou un groupe alkyle en $C_1$—$C_4$, ou un sel pharmaceutiquement acceptable de ce composé, en association avec un support pharmaceutiquement acceptable pour ce composé ou ce sel.

10. Composé répondant à la formule (I) dans laquelle $R^1$ a la signification définie dans la revendication 1 ou représente un groupe —$CO_2R^8$ dans lequel $R^8$ est un atome d'hydrogène ou un groupe alkyle en $C_1$—$C_4$, ou un sel pharmaceutiquement acceptable de ce composé en vue de l'utiliser pour le traitement des troubles cardiaques.